Europäisches Patentamt

(19) European Patent Office

Office européen des brevets

(11) Publication number : **0 346 785 B1**

# (12) EUROPEAN PATENT SPECIFICATION

(45) Date of publication of patent specification :
09.09.92 Bulletin 92/37

(51) Int. Cl.⁵ : **F16L 37/28, A61M 1/00**

(21) Application number : **89110542.1**

(22) Date of filing : **10.06.89**

(54) **Fluid cutoff device in a line.**

(30) Priority : **15.06.88 IT 2097788**

(43) Date of publication of application :
**20.12.89 Bulletin 89/51**

(45) Publication of the grant of the patent :
**09.09.92 Bulletin 92/37**

(84) Designated Contracting States :
**AT BE CH DE ES FR GB GR IT LI LU NL SE**

(56) References cited :
**EP-A- 0 113 871**
**WO-A-86/04130**
**AT-B- 362 637**
**US-A- 4 388 947**

(73) Proprietor : **DIDECO S.p.A.**
**Via Galilei 3**
**I-41037 Mirandola (Province of Modena) (IT)**

(72) Inventor : **Vescovini, Pietro**
**Via Artigiani, 10/12**
**I-41056 Medolla MO (IT)**

(74) Representative : **Modiano, Guido et al**
**MODIANO, JOSIF, PISANTY & STAUB**
**Modiano & Associati Via Meravigli, 16**
**I-20123 Milano (IT)**

EP 0 346 785 B1

Jouve, 18, rue Saint-Denis, 75001 PARIS

## Description

The invention relates to a fluid cutoff device in a line.

It is known that the problem of the fluid flow cutoff in a line often arises, and a very common case is for example that of bypass lines in medical devices; such lines, in fact, must normally be cutoff to prevent the flow of fluid, often constituted by blood, inside them, but in certain circumstances the cutoff must be releasable so that the fluid can flow.

Currently such lines are clamped using a clamp, but this system is not free from disadvantages which are particularly related to the lack of space normally available.

Alternatively, WO-A-8 604 130 shows a coupling device which includes complimentary-threaded male and female members. Each member contains a valve which is spring-biased into a closed position when the members are separated, or at least when they are relatively spaced apart. Upon a mutual closing rotation of the members, made possible by the complimentary threads, a stem of each valve which protrudes internally of the coupling device engages with an abutment to thereby open each valve.

The aim of the present invention is to provide a fluid cutoff device in a line which has a low cost and minimum bulk, and allows the cutoff of two branches of the line which lead to the device.

Within the scope of the proposed aim, an object of the invention is to provide a one-way device, i.e. a device which allows a one-way fluid flow.

The proposed aim and the above described object, are achieved by a fluid cutoff device in a line, according to the invention, as defined in the appended claims.

Further characteristics and advantages of the invention will become apparent from the description of some preferred but not exclusive embodiments of the invention, illustrated only by way of non-limitative example in the accompanying drawings, wherein:

figure 1 is a perspective view of the invention in the position in which the fluid flow is allowed;

figure 2 is a sectional view taken along the plane II-II of figure 1;

figure 3 is a perspective view of the invention in the fluid cutoff position;

figure 4 is a sectional view taken along the plane IV-IV of figure 3;

figure 5 is a sectional view of the invention, according to a first varied embodiment thereof, in open position;

figure 6 is a sectional view of the invention according to the embodiment of figure 5 in cutoff position;

figure 7 is a schematic view of some elements of the invention according to a second varied embodiment and with a shutter positioned according to a first possibility;

figure 8 is a schematic view of the same elements of figure 7 with the shutter positioned according to a second possibility;

figure 9 is a sectional view of the invention in open position with both shutters arranged according to the possibility illustrated in figure 7;

figure 10 is a sectional view of the invention in open position with one shutter arranged according to the possibility illustrated in figure 7 and one shutter arranged according to the possibility illustrated in figure 8.

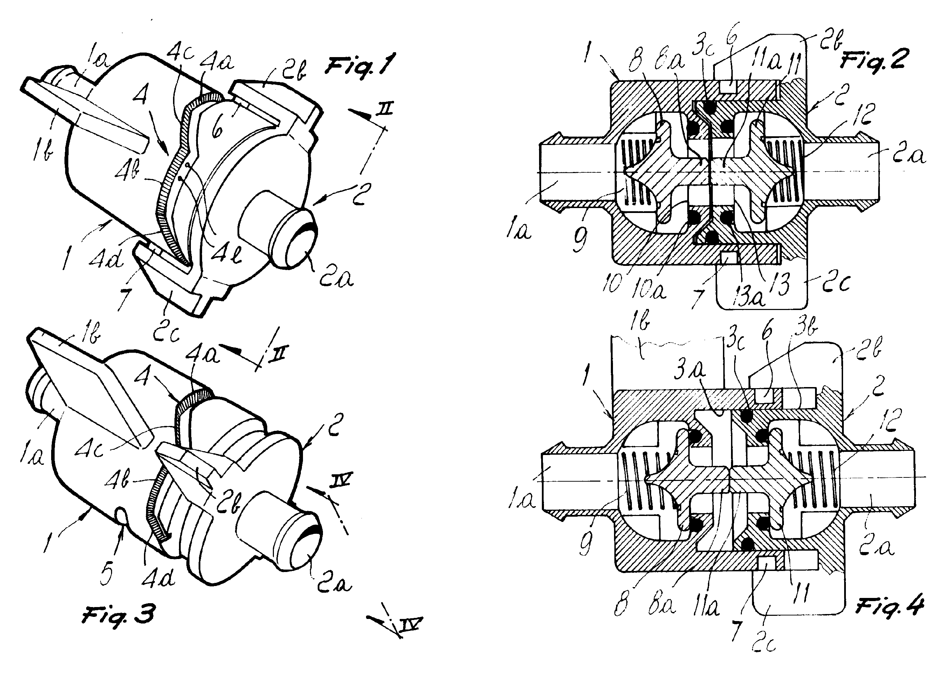

With reference to the above described figures 1, 2, 3, 4, the reference numerals 1 and 2 indicate the two interconnected bushes, respectively provided with connections 1a and 2a to two branches of a line and with grip elements constituted by the flap 1b and by the pair of flaps 2b, 2c; said bushes are in contact at the cylindrical surfaces respectively indicated by the reference numerals 3a, 3b in figure 4, and 3c indicates a sealing gasket which prevents any external contact of the fluid which flows through the device, and therefore ensures the seal and the sterility thereof.

The outer surface of the bush 1 has, in opposite positions, two identical grooves, the groove generally indicated by the reference numeral 4 being entirely visible in figures 1 and 3, while the groove indicated by the reference numeral 5 can be seen only in its end portion in figure 3.

Said groove 4 comprises the two circumferential portions 4a, 4b connected by the inclined portion 4c and the inclined portion 4d which is outwardly open; the groove 5 has, as mentioned, the same configuration.

A pin 6 which extends from the flap 2b of the bush 2 slides within the groove 4 and meets, in motion, an obstacle constituted by studs 4e which can be passed by elastic deformation and provide a slight locking action for an operator; similarly the pin 7 which extends from the flap 2c of said bush 2 slides in the groove 5. A shutter 8 is contained within the bush 1 and is pushed by the spring 9 against a sealing surface 10 which has a gasket 10a provided within said bush 1; in the same manner the bush 2 contains a shutter 11, which is pushed by a spring 12 against a sealing surface 13 having a gasket 13a provided within said bush 2.

Said shutters respectively have facing protrusions 8a and 11a to provide the operating functions described hereinafter.

In the position of figures 1 and 2 the two bushes are in the condition of maximum mutual approach, determined by the location of the pin 6 in the circumferential portion 4a of the groove 4 and of the pin 7 in the similar portion of the groove 5; in this condition the two shutters 8 and 11 are in mutual contact, pushed by the springs, and act as abutment elements for one another at the ends of the protrusions 8a and 11a and are well separated from contact with the sealing surfaces, respectively 10 and 13.

This is therefore the open position of the device, and the fluid can flow in both directions.

By rotating the bushes 1 and 2 until they move to the position of figures 3 and 4, with the pin 6 in abutment with the studs 4e, the bushes are mutually spaced due to the presence of the inclined portion 4c of the groove 4, and therefore the shutters 8 and 11, which are always pressed against one another by the action of the springs in the same position they occupied previously, make contact with their respective sealing surfaces.

This is therefore the cutoff position of the device, and it should be noted that each of the two branches of a line which reaches the device is cutoff by an individual shutter: the branch which reaches the bush 1 by the shutter 8, and the branch which reaches the bush 2 by the shutter 11.

This is the circumstance which allows, with a further relative rotation of the bushes from the described cutoff position, corresponding to the path of the pin 6 first in the circumferential portion and subsequently in the inclined portion 4d which is arranged outwardly open, and with a similar motion of the pin 7 in the groove 5, to cause the disconnection of one bush from the other and therefore to disconnect the two branches of the line without determining fluid losses.

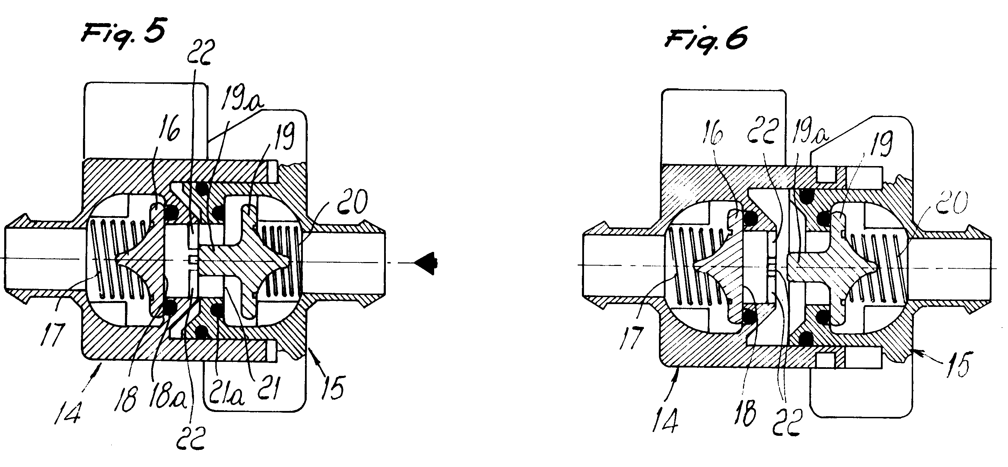

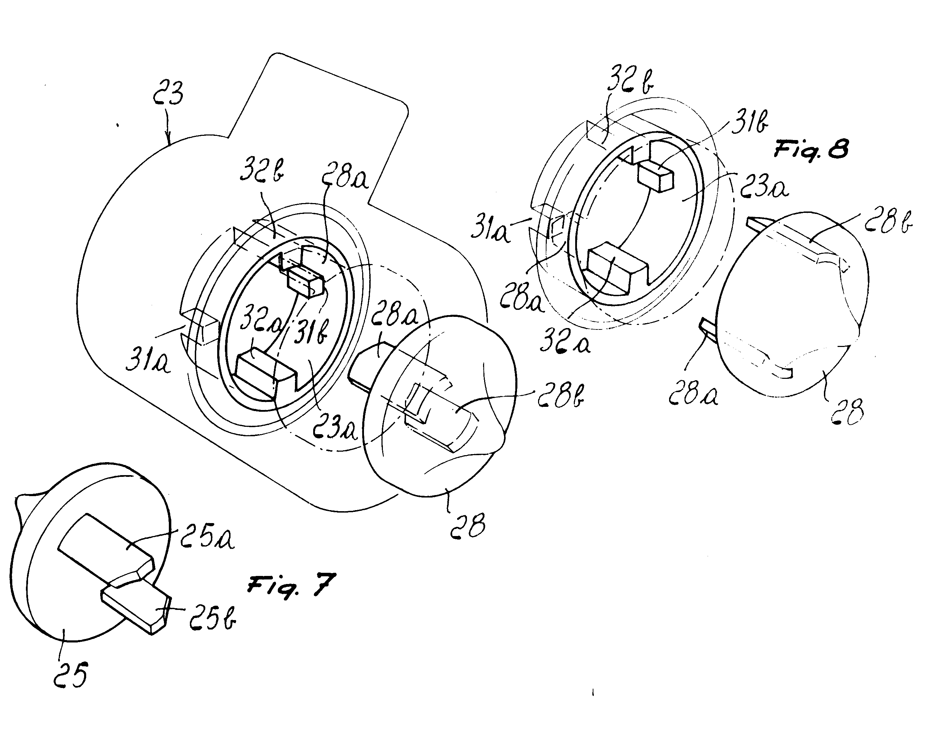

Figures 5 and 6 illustrate a first varied embodiment of the device which achieves, in the open position visible in figure 5, the condition of one-way flow in the direction of the arrow of said figure; figure 6 illustrates the cutoff condition.

The bushes 14 and 15 of this varied embodiment externally have a configuration which is identical to that of the previously described bushes 1 and 2, with the same grooves for the accommodation of the same pins, and therefore have identical characteristics of relative motion from a position of maximum approach shown in figure 5 to a spacing position shown in figure 6, up to the previously described possibility of separation.

The bush 14 contains the shutter 16, pushed by the spring 17 against the sealing surface 18 which has a gasket 18a, and said shutter 16 does not avail itself of elements adapted to make contact with abutment elements which can determine its loss of contact with the surface 18.

The bush 15 contains the shutter 19 which is pushed by the spring 20 against the sealing surface 21 which has a gasket 21a, and said shutter 19 has the protrusion 19a adapted to make contact with the ridges 22 provided within the bush 14.

In the open position shown in figure 5, with the bushes in the condition of maximum mutual approach, the protrusion 19a of the shutter 19 is in contact with the ridges 22 of the bush 14 which act as abutment elements, and therefore said shutter is pushed into a position of separation from its related sealing surface 21; the flow can occur only in the direction of the arrow, i.e. from the bush 15 towards the bush 14, by virtue of an appropriate setting of the spring 17 which yields, allowing the separation of the shutter 16 from the sealing surface 18, under the action of the thrust of the fluid against said shutter.

In the opposite direction the flow is obviously impossible. The mutual spacing of the bushes 14 and 15 moves the device to the cutoff position illustrated in figure 6: due to the loss of contact between the protrusion 19a of the shutter 19 and the ridges 22, said shutter, pushed by the spring 20, in fact makes contact with the sealing surface 21, and any flow is thus prevented.

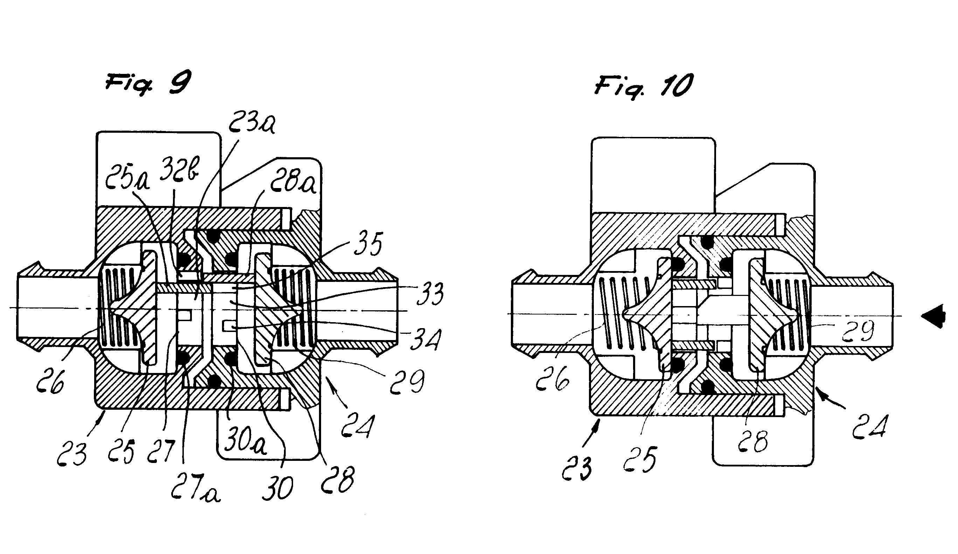

The bushes 23 and 24 of the second varied embodiment of the device, illustrated in figures 7, 8, 9, 10, also have an external configuration which is identical to that of the bushes 1 and 2 of the first described embodiment, with the same grooves for the accommodation of the same pins which guide said bushes from a position of maximum mutual approach shown in figures 9 and 10, to a spacing position which determines the cutoff of the fluid, up to the possibility of mutual separation.

The two bushes 23 and 24 contain two identical shutters, so as to be manufacturable with a single molding: the shutter indicated by the reference numeral 25, contained in the bush 23, provided with opposite flaps in the shape of a portion of a cylindrical surface 25a, 25b and pushed by the spring 26 against the sealing surface 27 provided with a gasket 27a; and the shutter indicated by the reference numeral 28, contained in the bush 24, provided with flaps 28a, 28b and pushed by the spring 29 against the sealing surface 30 provided with a gasket 30a.

The bush 23 comprises the cylindrical surface 23a having two pairs of opposite axial ridges with different longitudinal extension, i.e. the short ridges 31a,31b and the long ridges 32a, 32b; said ridges delimit, between each other, four portions of space which determine two different possibilities of accommodation of the flaps 25a,25b of the shutter 25; the flaps may in fact be accommodated in the spaces respectively between the ridges 31a, 32a and 32b,31b or in the spaces respectively between the ridges 31a,32b and 32a,31b.

The same situation occurs in the bush 24: said bush has a cylindrical surface 33 which has a pair of opposite axial short ridges 34 and a pair of opposite axial long ridges 35, and therefore also here, two different possibilities of accommodation of the flaps 28a,28b of the shutter 28 are provided.

Figure 7 illustrates in solid lines the shutter 28 in the first possibility of accommodation, in the position of mutual spacing of the bushes; when the bushes approach one another said shutter moves and rotates together with the bush 24 due to the traction exerted by the ridges of the surface 33 on the flaps thereof, and moves to the position shown in broken and dotted lines with the flap 28a against the long ridge 32b of the cylindrical surface 23a of the bush 23, and obviously with the flap 28b against the other long ridge 32a of said surface, thus providing the separation of said shutter 28 from the related sealing surface 30, as is clearly visible in figure 9.

The shutter 25 of said figure 9 is also arranged with its flaps between the ridges of the surface 23a of the bush 23 so as to make contact with said flaps in the position of maximum mutual approach of the bushes against the long ridges of the surface 33 of the bush 24, as shown in said figure 9 regarding the flap 25a against the ridge 35; in this case, therefore, the separation of said shutter 25 from its sealing surface 27 is also determined, and therefore a device has been provided which allows the flow of the fluid in both directions in its open position. But for both shutters there is a second possibility of accommodation in the related bushes.

Figure 8 indeed illustrates in solid lines the shutter 28 in its second possibility of accommodation, in the position of mutual spacing of the bushes; in this case, when the bushes approach, said shutter moves to the position shown in broken and dotted lines with the flap 28a at the short ridge 31a of the surface 23a of the bush 23, and obviously with the flap 28b at the other short ridge 31b, without making contact therewith, and therefore without determining separation of the shutter from its sealing surface.

It is evident that in this case the other shutter 25 must be caused to separate from its sealing surface in the position of maximum mutual approach of the bushes, thus providing a one-way device which allows the flow exclusively in one direction, from the bush 23 towards the bush 24.

If instead it is required to provide a one-way device with a single possible flow from the bush 24 to the bush 23 it is sufficient to arrange the elements as illustrated in figure 10: the shutter 28 is located so that its flaps make contact, in the position of maximum mutual approach of the bushes, with the long ridges of the bush 23, so as to separate from its sealing surface, and the shutter 25 is positioned with its flaps facing the short ridges of the bush 24, so as to not separate from its sealing surface.

Where technical features mentioned in any claim are followed by reference signs, those reference signs have been included for the sole purpose of increasing the intelligibility of the claims and accordingly, such reference signs do not have any limiting effect on the scope of each element identified by way of example by such reference signs.

## Claims

1. Device for the cutoff of fluid in a line, comprising two bushes (1,2;14,15;23,24) interconnected by means (4-7) adapted to determine their relative motion with at least one component in the direction of the axis, each having a connection (1a,2a) to a branch of the line and comprising a shutter (8,11;16,19;25,28) pushed by a spring (9,12;17, 20;26,29), in a position of mutual spacing of the two bushes, against a sealing surface (10,13; 18,21;27,30) comprised within said bush so as to achieve fluid cutoff in each branch of the line, at least one of the shutters being adapted to make contact with an abutment element (8a,11a;19a, 22;25a,25b,28a,28b) in the position of maximum mutual approach of the bushes so as to cause separation from the sealing surface, overcoming the action of the spring, characterized in that the outer surface of one bush (1) has at least one groove (4), comprising two circumferential portions (4a,4b) connected by a first inclined portion (4c) adapted to accommodate a pin (6) rigidly associated with the other bush (2) so as to provide a cam capable of determining the required relative motion between the two bushes upon a manual actuation thereof on adapted grip elements (1b,2b).

2. Device according to claim 1, characterized in that the means for connecting the two bushes further comprise a second outwardly arranged inclined portion (4d) which allows, upon a further mutual spacing motion of the bushes from the position of fluid cutoff in each branch of the line, the uncoupling of said bushes from one another.

3. Device according to one or more of the preceding claims, characterized in that the surface of contact (3a,3b) between the two bushes is cylindrical and has a sealing gasket (3c).

4. Device according to one or more of the preceding claims, characterized by the presence of an obstacle (41), which can be passed by elastic deformation, in the excursion of the pin (6) within said at least one groove (4), arranged at the mutual position of the bushes which determines the cutoff of the fluid in each of the two branches of the line.

5. Device according to one or more of the preceding claims, characterized in that the two shutters (8,11), are each comprised within a bush (1,2), and have facing protrusions (8a,11a) adapted to make mutual contact, so that each shutter acts as abutment element for the other, causing, in the position of maximum mutual approach of the two bushes, the separation of both said shutters from their respective sealing surfaces (10,13), with the possibility of fluid flow in both directions.

6. Device according to one or more of the preceding claims, characterized in that the shutter (19) provided in one (15) of the bushes has a protrusion (19a) adapted to make contact with ridges (22) provided within the other bush (14) so as to provide, in the position of maximum mutual approach of the bushes, the separation of said shutter from its related sealing surface (21), the other shutter (16) having no elements adapted to make contact with an abutment element and being therefore always pushed against its related sealing surface (18) by the action of a spring (17) calibrated so as to yield only upon the thrust of fluid against the shutter, thus providing a one-way device.

7. Device according to one or more of the preceding claims, characterized in that each shutter (25,28) has two opposite flaps (25a,25b,28a,28b) in the shape of a portion of a cylindrical surface adapted to be accommodated in one pair or the other of opposite portions of space defined in a cylindrical surface (23a,33) provided within the bush (23,24) which accommodates the shutter, due to the presence, on said cylindrical surface, of two pairs of opposite axial ridges (31a,31b,32a, 32b,34,35) with different longitudinal extension, said flaps of each shutter being, in one of the two possible arrangements of said shutter, adapted to make contact, during the step of mutual approach of the bushes, with the ridges (32a,32b,35) having greater axial extension provided in the bush accommodating the other shutter, with consequent separation of said shutter from the sealing surface, or to be positioned, in the other of the two possible arrangements, facing the ridges (31a, 31b,34) having shorter axial extension without making contact therewith and therefore without determining the separation of the shutter from its related sealing surface.

8. Device according to claim 7, characterized in that both shutters (25,28) are positioned so that their respective flaps make contact, in the position of maximum mutual approach of the bushes, with the ridges having greater axial extension of the opposite bush, so as to determine the possibility of flow of fluid in both directions.

9. Device according to claim 7, characterized in that one shutter (28) is positioned so that its flaps make contact, in the position of maximum mutual approach of the bushes, with the ridges having greater axial extension of the opposite bush, while the other shutter (25) is arranged so that its flaps face the ridges having shorter axial extension of said bush, so as to provide a one-way device.

**Patentansprüche**

1. Vorrichtung zur Absperrung eines Fluids in einer Rohrleitung, enthaltend zwei Hülsen (1, 2; 14, 15; 23, 24), die durch Mittel (4 bis 7) zur Bestimmung der Relativbewegung der Hülsen mit wenigstens einer Komponente in Richtung der Achse in Verbindung stehen, wobei jede dieser Hülsen einen Anschluß (1a, 2a) an einen Abschnitt einer Rohrleitung sowie ein Schließelement (8, 11; 16, 19; 25, 28) enthält, das in einer Position, in der die beiden Hülsen zueinander einen Abstand aufweisen, durch eine Feder (9, 12; 17, 20; 26, 29) gegeneine innerhalb der Hülse vorgesehene Dichtungsfläche (10, 13; 18, 21; 27, 30) gedrückt wird und hierdurch das Fluid in jedem Abschnitt der Rohrleitung absperrt, wobei wenigstens eines der Schließelemente entgegen der Federwirkung bei maximaler gegenseitiger Annäherung der Hülsen in Kontakt mit einem Anschlagelement (8a, 11a; 19a, 22; 25a, 25b, 28a, 28b) treten kann und hierdurch die Trennung von der Dichtungsfläche bewirkt, dadurch gekennzeichnet, daß die äußere Oberfläche einer Hülse (1) wenigstens eine Nut (4) aufweist, die zwei Umfangsbereiche (4a, 4b) enthält, die durch einen ersten geneigten Bereich (4c) zur Aufnahme eines Stiftes (6) verbunden sind, der fest mit der anderen Hülse (2) verbunden ist und dadurch einen Steuerkörper bildet sowie die gewünschte Relativbewegung zwischen den beiden Hülsen bei manueller Betätigung der Hülse mittels zweier geeigneter Griffelemente (1b, 2b) bestimmt.

2. Vorrichtung nach Anspruch 1, dadurch gekennzeichnet, daß die Einrichtung zur Verbindung der zwei Hülsen weiterhin einen zweiten, außen angeordneten, geneigten Bereich (4d) enthält, der das Lösen der Hülsen voneinander ermöglicht, wenn die Hülsen aus der Fluidabsperrposition in jedem Abschnitt der Rohrleitung in einen größeren Abstand zueinander geführt werden.

3. Vorrichtung nach wenigstens einem der vorangegangenen Ansprüche, dadurch gekennzeichnet, daß die Kontaktfläche (3a, 3b) zwischen den beiden Hülsen zylindrisch ist und eine Dichtungsfläche (3c) aufweist.

4. Vorrichtung nach wenigstens einem der vorangegangenen Ansprüche, dadurch gekennzeichnet, daß ein Widerstand (4e) in der Bewegungsbahn des Stiftes (6) in der wenigstens einen Nut (4) vorgesehen ist, der durch elastische Verformung überwunden werden kann und der in derjenigen Relativposition der Hülsen angeordnet ist, die die Absperrung des Fluids in jedem der beiden Abschnitte der Rohrleitung bestimmt.

5. Vorrichtung nach wenigstens einem der vorangegangenen Ansprüche, dadurch gekennzeichnet, daß die beiden Schließelemente (8, 11) innerhalb einer Hülse (1, 2) enthalten sind und einander gegenüberstehende Vörsprünge (8a, 11a) aufweisen, die den gegenseitigen Kontakt herstellen können, so daß jedes Schließelement als Anschlagelement für das andere wirkt, was in der Position der maximalen gegenseitigen Annäherung der beiden Hülsen die Trennung der beiden Hülsen von ihren jeweiligen Dichtungsflächen (10, 13) mit der Möglichkeit eines Fluidflusses in beiden Richtungen bewirkt.

6. Vorrichtung nach wenigstens einem der vorangegangenen Ansprüche, dadurch gekennzeichnet, daß das Schließelement (19), das in einer (15) der Hülsen angeordnet ist, einen Vorsprung (19a) aufweist, der Vorsprünge (22) berühren kann, die innerhalb der anderen Hülse (14) vorgesehen sind, um in der Position der maximalen gegenseitigen Annäherung der Hülsen die Trennung des Schließelements von der zugehörigen Dichtungsfläche (21) zu bewirken, wobei das andere Schließelement (16) keine Elemente aufweist, die ein Anschlagelement berühren können, und daher immer gegen die zugehörige Dichtungsfläche (18) durch die Wirkung einer Feder (17) gedrückt wird, die so eingestellt ist, daß sie nur dem Druck des Fluids gegen das Schließelement nachgibt, wodurch eine Ein-Wege-Richtung vorliegt.

7. Vorrichtung nach wenigstens einem der vorangegangenen Ansprüche, dadurch gekennzeichnet, daß jedes Schließelement (25, 28) zwei einander gegenüberliegende teilzylinderische Laschen (25a, 25b, 28a, 28b) aufweist, die in das eine oder das andere Paar von einander gegenüberliegenden Raumabschnitten eingreifen können, die in einer innerhalb der Hülse (23, 24) vorgesehenen zylindrischen Oberfläche (23a, 33) ausgebildet sind, die das Schließelement aufgrund des Vorhandenseins von zwei auf der zylinderischen Oberfläche angeordneten Paaren von einander axial gegenüberliegenden Vorsprüngen (31a, 31b, 32a, 32b, 34, 35) mit unterschiedlichen Längen unterbringt, wobei die Laschen jedes Schließelements in der einen der beiden möglichen Stellungen des Schließelements während der gegenseitigen Annäherung der Hülsen die Vorsprünge (32a, 32b, 35) berühren können, die eine größere axiale Erstreckung aufweisen und in der das andere Schließelement aufnehmenden Hülse vorgesehen sind, wodurch das Schließelement von der zugehörigen Dichtungsfläche getrennt wird, und wobei die Laschen in der anderen Stellung den Vorsprüngen (31a, 31b, 34) kürzerer axialer Erstreckung gegenüberliegen, ohne diese zu berühren, wodurch die Trennung des Schließelements von der zugehörigen Dichtungsfläche aufgehoben wird.

8. Vorrichtung nach Anspruch 7, dadurch gekennzeichnet, daß beide Schließelemente (25, 28) so positioniert sind, daß ihre jeweiligen Laschen in der Position der maximalen gegenseitigen Annäherung der Hülsen die der anderen Hülse zugehörenden Vorsprünge mit größerer axialer Erstreckung berühren, und so die Möglichkeit des Flüssigkeitsflusses in beiden Richtungen bestimmen.

9. Vorrichtung nach Anspruch 7, dadurch gekennzeichnet, daß ein Schließelement (28) so positioniert ist, daß seine Laschen in der Position der maximalen gegenseitigen Annäherung der Hülsen die der anderen Hülse zugehörigen Vorsprünge mit größerer axialer Erstreckukng berühren, während das andere Schließelement (25) so angeordnet ist, daß seine Laschen den der anderen Hülse zugehörenden Vorsprüngen mit kürzerer axialer Erstreckung gegenüberliegen, um so eine Ein-Wege-Vorrichtung zu erstellen.

## Revendications

1. Dispositif de coupure de fluide dans une conduite, comportant deux manchons (1, 2; 14, 15; 23, 24) interconnectés par des moyens (4 à 7) prévus pour déterminer leur déplacement relatif avec au moins une composante dans la direction de l'axe, ayant chacun un raccord (1a, 2a) pour une branche de la conduite et comportant un obturateur (8, 11; 16, 19; 25, 28) poussé par un ressort (9, 12; 17, 20; 26, 29), dans une position d'espacement mutuel des deux manchons, contre une surface d'étanchéité (10, 13; 18, 21; 27, 30) comprise à l'intérieur dudit manchon de façon à réaliser la coupure de fluide dans chaque branche de la conduite, au moins un des obturateurs étant prévu pour réaliser un contact avec un élément de butée (8a, 11a; 19a, 22; 25a, 25b, 28a, 28b) dans la position d'approche mutuelle maximum des manchons de façon à entraîner une séparation de la surface d'étanchéité en surmontant l'action du ressort, caractérisée en ce que la surface extérieure d'un manchon (1) possède au moins une rainure (4), comportant deux parties circonférentielles (4a, 4b) reliés par une première partie inclinée (4c) prévue pour recevoir un ergot (6) associé rigidement à l'autre manchon (2) de façon à procurer une came capable de déterminer le déplacement relatif requis entre les deux manchons lors d'un actionnement manuel de ceux-ci sur des éléments de saisie adaptés (1b, 2b).

2. Dispositif selon la revendication 1, caractérisé en ce que les moyens destinés à relier les deux manchons comportent en outre une deuxième partie inclinée disposée extérieurement (4d) qui permet, lors d'un autre déplacement d'espacement mutuel des manchons à partir de la position de coupure de fluide dans chaque branche de la conduite, le désaccouplement desdits manchons.

3. Dispositif selon une ou plusieurs des revendications précédentes, caractérisé en ce que la surface de contact (3a, 3b) entre les deux manchons est cylindrique et possède un joint d'étanchéité (3c).

4. Dispositif selon une ou plusieurs des revendications précédentes, caractérisé par la présence d'un obstacle (41), qui peut être dépassé par déformation élastique, dans le passage de l'ergot (6) à l'intérieur de ladite rainure (4), disposé dans la position mutuelle des manchons qui détermine la coupure du fluide dans chacune des deux branches de la conduite.

5. Dispositif selon une ou plusieurs des revendications précédentes, caractérisé en ce que les deux obturateurs (8, 11) sont compris chacun à l'intérieur d'un manchon (1, 2) et possèdent des saillies se faisant face (8a, 11a) prévues pour réaliser un contact mutuel, de telle sorte que chaque obturateur agit comme élément de butée pour l'autre, amenant, dans la position d'approche mutuelle maximum des deux manchons, la séparation des deux obturateurs de leurs surfaces d'étanchéité respectives (10, 13), avec la possibilité d'écoulement de fluide dans les deux directions.

6. Dispositif selon une ou plusieurs des revendications précédentes, caractérisé en ce que l'obturateur (19) prévu dans un (15) des manchons possède une saillie (19a) prévue pour réaliser un contact avec des nervures (22) prévues à l'intérieur de l'autre manchon (14) de façon à procurer, dans la position d'approche mutuelle maximum des manchons, la séparation dudit obturateur de sa surface d'étanchéité associée (21), l'autre obturateur (16) n'ayant pas d'éléments prévus pour réaliser un contact avec un élément de butée et étant par conséquent toujours poussée contre sa surface d'étanchéité associé (18) par l'action d'un ressort (17) étalonné de façon à s'enfoncer uniquement sous la poussée de fluide contre l'obturateur, procurant ainsi un dispositif anti-retour.

7. Dispositif selon une ou plusieurs de revendications précédentes, caractérisé en ce que chaque obturateur (25, 28) possède deux volets opposés (25a, 25b, 28a, 28b) sous la forme d'une partie d'une surface cylindrique prévus pour être reçus dans une paire ou dans les autres parties opposées de l'espace défini dans une surface cylindrique (23a, 33) prévues à l'intérieur du manchon (23, 24) qui reçoit l'obturateur, du fait de la présence, sur ladite surface cylindrique, de deux paires de nervures axiales opposées (31a, 31b, 32a, 32b, 34, 35) avec une extension longitudinale différente, lesdits volets de chaque obturateur étant, dans l'un des deux agencements possibles dudit obturateur, prévu pour réaliser un contact, pendant l'étape d'approche mutuelle des manchons, avec les nervures (32a, 32b, 35) ayant une extension axiale supérieure prévues dans le manchon recevant l'autre obturateur, avec une séparation consécutive dudit obturateur de la surface d'étanchéité, ou pour être positionnés, dans l'autre des deux agencements possibles, face aux nervures (31a, 31b, 34) ayant une extension axiale plus courte sans réaliser le contact avec celles-ci et sans déterminer par conséquent la séparation de l'obturateur de sa surface d'étanchéité associé.

8. Dispositif selon la revendication 7, caractérisé en ce que les deux obturateurs (25, 28) sont positionnés de telle sorte que leurs volets respectifs réalisent un contact, dans la position d'approche mutuelle maximum des manchons, avec les nervures ayant une extension axiale plus grande du manchon opposé, de façon à déterminer la possibilité d'écoulement de fluide dans les deux directions.

9. Dispositif selon la revendication 7, caractérisé en ce qu'un obturateur (28) est positionné de telle sorte que ses volets réalisent un contact, dans la position d'approche mutuelle maximum des manchons, avec les nervures ayant une extension axiale supérieure du manchon opposé, alors que l'autre obturateur (25) est prévu de telle sorte que ses volets font face aux nervures ayant une extension axiale plus courte dudit manchon, de façon à procurer un dispositif anti-retour.

Fig.1

Fig.2

Fig.3

Fig.4

Fig.6

Fig.5

Fig. 7

Fig. 8

EP 0 346 785 B1

Fig. 9

Fig. 10

EP 0 346 785 B1